# EUROPEAN PATENT APPLICATION

(11) **EP 2 266 975 A1**
(43) Date of publication of application: **29.12.2010**
(21) Application number: 09162731.5
(22) Date of filing: 15.06.2009
(51) Int. Cl.: C07D 405/04, A61K 31/4025, A61P 25/00

(54) **1-(2-alkyl-2,3-dihydro-benzofuran-4-yl)-pyrrolidin-3-ylamine acyl compounds**

(71) Applicant: Ferrer Internacional, S.A., 08028 Barcelona (ES)
(72) Inventor: Falco, Josep Lluis, 08004, Barcelona (ES); Palomer, Albert, 08014, Barcelona (ES)
(74) Representative: Reitstötter - Kinzebach

(57) **Abstract**

This invention provides new 1-(2-alkyl-2,3-dihydro-benzofuran-4-yl)-pyrrolidin-3-ylamine acyl compounds, their use for the treatment or prevention of melatoninergic disorders and its compositions.

## Description

### FIELD OF THE ART

The invention belongs to the field of compounds with high affinity on melatonin receptors, specifically 1-(2-alkyl-2,3-dihydro-benzofuran-4-yl)-pyrrolidin-3-ylamine compounds, and more specifically 1-(2-alkyl-2,3-dihydro-benzofuran-4-yl)-pyrrolidin-3-ylamine acyl compounds.

### STATE OF THE ART

Insomnia is a very common disorder, affecting from 20 to 40% of the adult population, with an increasing incidence in the elderly. Insomnia can be due to many causes. One of them is the disturbance of the normal regulating sleep-wake cycles. This asynchrony can result in pathological changes. A potential therapeutic treatment to alleviate this effect consists in resynchronizing the sleep-wake cycles by modulating the melatonergic system (Sun L. Q. et al., Bioorganic & Medicinal Chemistry Letters 2005, 15, 1345-49).

Melatonin is a hormone secreted by the pineal gland in mammals being produced at night to aid the body in regulating sleep-wake cycles, and it is also responsible for the photoperiodic information, and for the modulation of the retina physiology. The secretion of melatonin in humans occurs simultaneously to nocturnal sleep, and the increase of melatonin levels is correlated with the increase of somnolence at nightfall. The amount of melatonin the body produces decreases with age, which may explain why the elderly suffer from insomnia more frequently than the general population. The therapeutic uses of melatonin in humans embrace the treatment of sleep delay syndrome and jetlag, including the treatment of nocturnal workers, and as hypnotic by itself. However, its short half-life (minutes) limits its therapeutic use. The synthesis of melatonin and the nocturnal secretion thereof are controlled by the supraquiasmatic nucleus, and are synchronized by the environmental light (Osamu Uchikawa et al., J. Med. Chem. 2002, 45, 4222-39; Pandi-Perumal et al., Nature Clinical Practice 2007, 3 (4), 221-228).

Melatonin receptors have been classified as MT1, MT2, and MT3, on the basis of pharmacological profiles. MT1 receptor is localized in the hypothalamic Central Nervous System, while MT2 receptor is distributed in the Central Nervous System and in the retina. The presence of MT1 and MT2 receptors has been disclosed also at peripheral level. MT1 and MT2 receptors are involved in a large number of conditions, such as depression, stress, sleep disorders, anxiety, seasonal affective disorders, cardiovascular pathology, pathology of the digestive system, insomnia or fatigue due to jetlag, schizophrenia, panic attacks, melancholia, appetite disorders, obesity, insomnia, psychotic disorders, epilepsy, diabetes, Parkinson's disease, senile dementia, disorders associated with normal or pathological ageing, migraine, memory loss, Alzheimer's disease and cerebral circulation disorders. The MT3 receptor has been characterized as homologous to the quinone reductase-2 (QR2) enzyme. MT1 y MT2 receptors are coupled to G-Protein-coupled Receptor (GPCR) whose stimulation by an agonist produces a decrease in the adenylate cyclase activity and a subsequent decrease in the intracellular cAMP.

Synthetic melatonin receptors agonists have been object of intense research in recent years. In addition to its first use for insomnia, they may have potential application in the synchronization of disturbed circadian rhythms, sleep disturbances in the elderly, seasonal depression and jetlag, among many others. Furthermore, it has been shown that melatonin receptor agonists do not induce any of the hypothermic, hypotensive or bradycardic effects caused by melatonin in humans.

Ramelteon, N-[2-[(8*S*)-1,6,7,8-tetrahydro-2*H*-indeno[5,4-*b*]furan-8-yl)ethyl] propionamide (US 6034239, Examples 19 and 20) has been the first melatonin receptors agonist approved by the US Food and Drug Administration (FDA) for therapeutic use in insomnia with no time limitation.

The approval of ramelteon represents an important milestone for the proof of concept of the melatonin target, and has opened new possibilities for research. However, there is an increasing need for new compounds with improved properties, such as more sustained plasma levels in order to provide more efficient treatments of conditions mediated by melatonin receptors.

Patent EP1189900B1 and Sun L. Q. et al., Bioorganic & Medicinal Chemistry Letters (2003), 13(24), 4381-4384, disclose a number of 1-(2,3-dihydro-benzofuran-4-yl)-pyrrolidin-3-ylamine compounds having melatonergic activity.

The compounds of the present invention differ therefrom by the specific alkyl substituent at the 2 position of the 2,3-dihydro-benzofuran ring. The compounds of the present invention are useful in the treatment and prevention of all those diseases that are mediated by melatonin receptors. Some non-limitative examples of melatoninergic disorders are depression, stress, sleep disorders, anxiety, seasonal affective disorders, cardiovascular pathologies, pathology of the digestive system, insomnia or fatigue due to jetlag, schizophrenia, panic attacks, melancholia, appetite disorders, obesity, insomnia, psychotic disorders, epilepsy, diabetes, Parkinson's disease, senile dementia, disorders associated with normal or pathological ageing, migraine, memory loss, Alzheimer's disease and cerebral circulation disorders.

### DETAILED DESCRIPTION OF THE INVENTION

The present invention relates to 1-(2-alkyl-2,3-dihydro-benzofuran-4-yl)-pyrrolidin-3-ylamine acyl compounds of general formula I 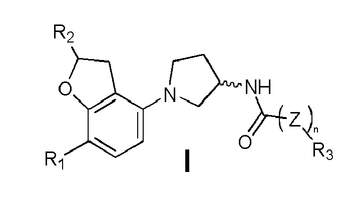 in free or pharmaceutically acceptable salt, solvate, hydrate, and enantiomeric form, wherein:
R₁ is selected from the group consisting of hydrogen and halogen;
R₂ is selected from the group consisting of linear or branched (1-6C)alkyl;
R₃ is selected from the group consisting of linear or branched (1-6C)alkyl, optionally substituted by 1 to 3 F atoms, and (3-6C)cycloalkyl;
Z is selected from the group consisting of NH or O; and
n is 0 or 1.

Pharmaceutically acceptable salts are those that may be administered to a patient, such as a mammal (e.g. salts with acceptable safety in mammals for a given dosing regimen). Such salts may be obtained from pharmaceutically acceptable inorganic and organic bases and from pharmaceutically acceptable inorganic and organic acids. The salts obtained from pharmaceutically acceptable inorganic bases include ammonium, calcium, copper, ferric and ferrous salts, lithium, magnesium, manganic and manganous salts, potassium, sodium, zinc salts and the like. Especially preferred are the ammonium, calcium, magnesium, potassium and sodium salts. The salts obtained from pharmaceutically acceptable organic bases include primary, secondary and tertiary amine salts, including substituted amines, cyclic amines, natural amines and the like, such as arginine, betaine, caffeine, choline, N,N'-dibenzylethylendiamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. The salts obtained from pharmaceutically acceptable acids include acetic, ascorbic, benzene sulphonic, benzoic, camphosulphonic, citric, ethanesulphonic, edisylic, fumaric, gentisic, gluconic, glucuronic, glutamic, hippuric, hydrobromic, hydrochloric, isethionic, lactic, lactobionic, maleic, malic, mandelic, methanesulphonic, mucic, naphthalenesulphonic, naphthalene-1,5-disulphonic, naphthalene-2,6-disulphonic, nicotinic, nitric, orotic, pamoic, pantothenic, phosphoric, succinic, sulphuric, tartaric, p-toluenesulphonic, xinafoic and the like. Particularly preferred are citric, hydrobromic, hydrochloric, isethionic, maleic, naphthalene-1,5-disulphonic, phosphoric, sulphuric and tartaric acids.

The specific compounds of formula I are chosen from the group consisting of:
*(i)* N-((3*S*)-1-(7-fluoro-2-methyl-2,3-dihydrobenzofuran-4-yl)pyrrolidin-3-yl)acetamide;
*(ii)* 2,2,2-trifluoro-N-((3*S*)-1-(7-fluoro-2-methyl-2,3-dihydrobenzofuran-4-yl)pyrrolidin-3-yl)acetamide;
*(iii)* N-((3*S*)-1-(7-fluoro-2-methyl-2,3-dihydrobenzofuran-4-yl)pyrrolidin-3-yl)propionamide;
*(iv)* 2-fluoro-N-((3*S*)-1-(7-fluoro-2-methyl-2,3-dihydrobenzofuran-4-yl)pyrrolidin-3-yl)propanamide;
*(v)* N-((3*S*)-1-(7-fluoro-2-methyl-2,3-dihydrobenzofuran-4-yl)pyrrolidin-3-yl)butyramide;
*(vi)* N-((3*S*)-1-(7-fluoro-2-methyl-2,3-dihydrobenzofuran-4-yl)pyrrolidin-3-yl)isobutyramide;
*(vii)* N-((3*S*)-1-(7-fluoro-2-methyl-2,3-dihydrobenzofuran-4-yl)pyrrolidin-3-yl)cyclopropanecarboxamide;
*(viii)* N-((3*S*)-1-(7-fluoro-2-methyl-2,3-dihydrobenzofuran-4-yl)pyrrolidin-3-yl)pentanamide;
*(ix)* N-((3*S*)-1-(7-fluoro-2-methyl-2,3-dihydrobenzofuran-4-yl)pyrrolidin-3-yl)-2-methylbutanamide;
*(x)* methyl (3*S*)-1-(7-fluoro-2-methyl-2,3-dihydrobenzofuran-4-yl)pyrrolidin-3-ylcarbamate;
*(xi)* ethyl (3*S*)-1-(7-fluoro-2-methyl-2,3-dihydrobenzofuran-4-yl)pyrrolidin-3-ylcarbamate;
*(xii)* 1-ethyl-3-((3*S*)-1-(7-fluoro-2-methyl-2,3-dihydrobenzofuran-4-yl)pyrrolidin-3-yl)urea;
*(xiii)* N-((3*S*)-1-(2-methyl-2,3-dihydrobenzofuran-4-yl)pyrrolidin-3-yl)acetamide;
*(xiv)* 2,2,2-trifluoro-N-((3*S*)-1-(2-methyl-2,3-dihydrobenzofuran-4-yl)pyrrolidin-3-yl)acetamide;
*(xv)* N-((3*S*)-1-(2-methyl-2,3-dihydrobenzofuran-4-yl)pyrrolidin-3-yl)propionamide;
*(xvi)* N-((3*S*)-1-(2-methyl-2,3-dihydrobenzofuran-4-yl)pyrrolidin-3-yl)isobutyramide;
*(xvii)* N-((3*S*)-1-(2-methyl-2,3-dihydrobenzofuran-4-yl)pyrrolidin-3-yl)cyclopropanecarboxamide;
*(xviii)* 3-methyl-N-((3*S*)-1-(2-methyl-2,3-dihydrobenzofuran-4-yl)pyrrolidin-3-yl)butanamide;
(xix) N-((3*S*)-1-(2-ethyl-2,3-dihydrobenzofuran-4-yl)pyrrolidin-3-yl)acetamide;
*(xx)* N-((3*S*)-1-(2-ethyl-2,3-dihydrobenzofuran-4-yl)pyrrolidin-3-yl)propionamide; and
*(xxi)* N-((3*S*)-1-(2-propyl-2,3-dihydrobenzofuran-4-yl)pyrrolidin-3-yl)propionamide; and
   the pharmaceutically acceptable salt, solvate, hydrate, and enantiomeric forms thereof.

Table 1 shows the meaning of the substituents for each compound:

**Table 1**

| ***Compound*** | ***R₁*** | ***R₂*** | ***R₃*** | ***Z*** | ***n*** |
|---|---|---|---|---|---|
| **1** | F | Me | Me | --- | 0 |
| **2** | F | Me | CF₃ | --- | 0 |
| **3** | F | Me | Ethyl | --- | 0 |
| **4** | F | Me | CHFCH₃ | --- | 0 |
| **5** | F | Me | n-Pr | --- | 0 |
| **6** | F | Me | i-Pr | --- | 0 |
| **7** | F | Me | c-Pr | --- | 0 |
| **8** | F | Me | n-Bu | --- | 0 |
| **9** | F | Me | i-Bu | --- | 0 |
| **10** | F | Me | Me | ○ | 1 |
| **11** | F | Me | Et | ○ | 1 |
| **12** | F | Me | Et | NH | 1 |
| **13** | H | Me | Me | --- | 0 |
| **14** | H | Me | CF₃ | --- | 0 |
| **15** | H | Me | Et | --- | 0 |
| **16** | H | Me | i-Pr | --- | 0 |
| **17** | H | Me | c-Pr | --- | 0 |
| **18** | H | Me | i-Bu | --- | 0 |
| **19** | H | Et | Me | --- | 0 |
| **20** | H | Et | Et | --- | 0 |
| **21** | H | Pr | Et | --- | 0 |

From formula **I** it is evident that the compounds of this invention have at least two asymmetric carbon atoms in their structure, namely the carbon atom bearing the R₂ substituent, and position 3 of the pyrrolidine ring. The absolute configuration of this centre is indicated by the stereochemical descriptors R and S. Unless otherwise mentioned or indicated, the chemical designation of compounds denotes the mixture of all possible stereochemically isomeric forms, said mixtures containing all diastereomers and enantiomers of the basic molecular structure. Stereochemically isomeric forms of the compounds of formula **I** are obviously intended to be embraced within the scope of the invention.

Another aspect of the present invention is to provide the use of a specific compound from Table 1 to prepare a medicinal product for the treatment or prevention of melatoninergic disorders. Said melatoninergic disorders are chosen from depression, stress, sleep disorders, anxiety, seasonal affective disorders, cardiovascular pathologies, digestive system pathologies, insomnia or fatigue due to jet lag, schizophrenia, panic attacks, melancholia, appetite disorders, obesity, insomnia, psychotic diseases, epilepsy, diabetes, Parkinson's disease, senile dementia, disorders associated to normal or pathological aging, migraine, memory loss, Alzheimer's disease and brain circulation disorders.

Another aspect of the present invention is to provide pharmaceutical compositions comprising a specific compound from Table 1 and one or more pharmaceutically acceptable excipients.

Another aspect of the present invention is to provide the use of said pharmaceutical compositions in the preparation of a medicinal product for the treatment or prevention of melatoninergic disorders. Said melatoninergic disorders are chosen from depression, stress, sleep disorders, anxiety, seasonal affective disorders, cardiovascular pathologies, digestive system pathologies, insomnia or fatigue due to jet lag, schizophrenia, panic attacks, melancholia, appetite disorders, obesity, insomnia, psychotic diseases, epilepsy, diabetes, Parkinson's disease, senile dementia, disorders associated to normal or pathological aging, migraine, memory loss, Alzheimer's disease and brain circulation disorders.

Another aspect of the present invention is to provide a method of treating or preventing melatoninergic diseases that comprises administering an effective amount of a specific compound from Table 1 to a patient.

The compounds of the general formula **I** may be prepared by
i) a) reacting a compound of formula X, 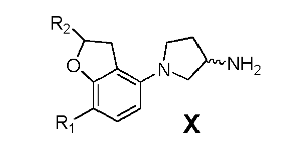 wherein R₁ and R₂ are as defined herein, with a coupling agent selected from the group consisting of: a) an acyl chloride of formula R₃-COCl; b) a chloroformate of formula R₃-O-COCl; and c) an isocyanate of formula R₃-N=CO; wherein R₃ is as described herein.
ii) recovering the resultant compound of formula **I** in free or pharmaceutically acceptable salt, solvate, hydrate, and enantiomeric form.

The compounds of the present invention can be prepared from 2-fluoro-4-nitro-phenol, (for R₁=F), according to Scheme 1. The compounds of the present invention when R₁=H can be prepared from commercially available N-(3-methoxyphenyl)pivalamide (**IV**, R₁=H) according to the same scheme, Final step is generalized for all meanings of R₁, R₂, Z, n and R₃ as described herein.

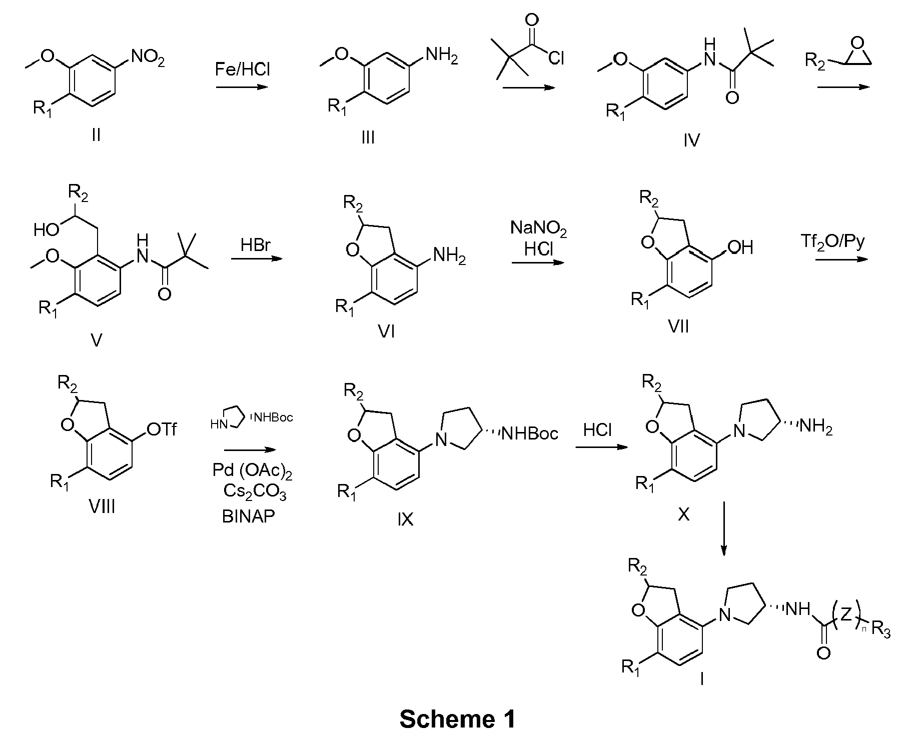

Stating from 1-fluoro-2-methoxy-4-nitrobenzene **II** by reduction of the nitro group and protection of the aniline formed, compound **IV** is obtained. The anion generated from **IV** and BuLi reacts with the required epoxide to form the ethylhydroxy derivative **V**. One-pot methoxy cleavage and ring-closing ether formation generates the benzofurane derivative **VI**. Diazonium salt mediated reaction generates the phenol derivative **VII** that undergoes activation to the triflate derivative **VIII.** This triflate derivative reacts by Buchwald reaction to the intermediated **IX** which is deprotected with HCl to generate the amine **X**. Finally, **X** is reacted with several coupling agents to yield final products of general structure **I**.

Appropriate coupling agents comprise: a) acyl chlorides of formula R₃-COCI, b) chloroformates of formula R₃-O-COCl, and c) isocyanates of formula R₃-N=C=O, thus providing compounds of formula **I**, wherein R₃-(Z)ₙ-represents a) R₃, as a linear or branched (1-6C)alkyl, (3-6C)cycloalkyl, CHFCH₃, and CF₃; b) OR₃, wherein R₃ is as described herein; and c) NHR₃, wherein R₃ is as described herein, respectively.

Useful processes for recovering the resultant compounds in step (ii) include conventional methods known to the person skilled in the art such as crystallization and chromatographic processes, resolution of racemic forms by chromatographic separation using a chiral stationary phase, and also processes involving fractional crystallization. This can, in particular, involve the separation of individual enantiomers, for example, diastereoisomeric salts formed with chiral acids, for instance (+)-tartaric acid, (-)-tartaric acid, or (+)-10-camphorsulfonic acid.

The compounds of formula **I** show the surprising advantage of being maintained at more sustained plasma levels and thus being long acting. This can be evidenced, for instance, by measuring the plasma levels after oral administration to rats at 15 and 60min after oral administration, resulting in lower ratio values for the compounds of the present invention. Thus the compounds of formula I, **characterized in that** R₂ is an alkyl radical show more sustained plasma levels than prior art compounds (EP1189900B1), wherein R₂ is hydrogen. Hence, the compounds of the invention provide longer sleep duration. Consequently, the present compounds allow for a more efficient therapy: the slow elimination facilitates maintaining a stable plasma concentration at a non-toxic, effective level and the prolonged effect may be expected to result in better compliance of the subject to be treated with the prescribed medication.

Pharmaceutical compositions comprising compounds of the present invention include those that are adequate for oral, rectal and parenteral administration (including the subcutaneous, intramuscular and intravenous routes), although the most suitable route will depend on the nature and seriousness of the pathology being treated. The preferred administration route for the compounds of the present invention is frequently the oral route.

The active ingredients can be mixed with one or more pharmaceutical excipients following conventional pharmaceutical techniques for formulation. Several excipients can be used according to the pharmaceutical form to be prepared. Liquid oral compositions (such as, for example, suspensions, solutions, emulsions, aerosols and mouthwashes) may use, for example, water, glycols, oils, alcohols, flavour enhancers, preservatives, colorants and the like. Solid oral compositions use, for example, starches, sugars (such as, for example, lactose, sucrose and sorbitol) celluloses (such as, for example, hydroxypropyl cellulose, carboxymethyl cellulose, ethyl cellulose and microcrystalline cellulose), talc, stearic acid, magnesium stearate, dicalcium phosphate, rubbers, copovidone, surfactants such as sorbitan monooleate and polyethyleneglycol, metallic oxides (such as, for example, titanium dioxide and ferric oxide) and other pharmaceutical diluents such as water. Homogeneous preformulations are thus formed containing the compounds of the present invention.

In the case of the preformulations the compositions are homogeneous, such that the active ingredient is dispersed uniformly in the composition, which can therefore be divided in equal unit doses such as tablets, coated tablets, powders and capsules.

Tablets and capsules are most advantageous oral forms due to their ease of administration. Tablets can be coated using aqueous or nonaqueous conventional techniques if so desired. A large variety of materials can be used to form the coating. Such materials include a large number of polymeric acids and their mixtures with other components such as, for example, shellac, cetyl alcohol and cellulose acetate.

Liquid forms in which the compounds of the present invention can be incorporated for oral or injectable administration include aqueous solutions, capsules filled with fluid or gel, syrups with flavour enhancers, aqueous suspensions in oil and emulsions flavoured with edible oils such as, for example, cottonseed oil, sesame oil, coconut oil or peanut oil, as well as mouthwashes and similar pharmaceutical carriers. Suitable dispersing or suspension agents for the preparation of aqueous suspensions include synthetic and natural gums such as tragacanth, Acacia, alginates, dextranes, sodium carboxymethylcellulose, methylcellulose, polyethyleneglycol, polyvinylpyrrodidone or gelatin.

A suitable dosage range to be used is a total daily dose from 0.1 to 500 mg approximately, more preferably from 1 mg to 100 mg, either in a single administration or in separate doses if necessary.

### EMBODIMENTS OF THE INVENTION

The present invention is additionally illustrated by means of the following examples, which do not intent to limit the scope thereof.

### Preparation of intermediates of formula IV

### N-(4-fluoro-3-methoxyphenyl)pivalamide

### Step 1: 4-fluoro-3-methoxyaniline

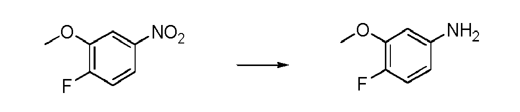

Nitro compound **II** (6.5 g, 0.038 mol) and iron powder (10.6 g, 0.19 mol) were suspended in ethanol (90 ml) at 0°C. HCl conc. (120 ml) was added drop wise to the reaction mixture. The reaction mixture was stirred at 20°C for 18hr. The reaction mixture was then filtered through celite and washed repeatedly with ethanol. Then ethanol was concentrated and the residue was basified with solid Na₂CO₃. Then it was extracted with ethyl acetate and washed with water and brine. The organic layer was dried over Na₂SO₄ and concentrated in vacuum to get the product (5 g, Y= 93%). HPLC-MS: Purity 97%, M+1= 142.1

### Step 2: N-(4-fluoro-3-methoxyphenyl)pivalamide

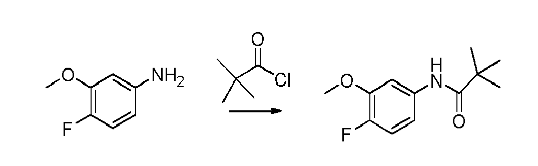

Compound **III** (8.9 g, 0.063 mol) was dissolved in dry dichloromethane (DCM) (90 ml). Pyridine (10.2 ml) was added drop wise to the reaction mixture at 20°C. Then pivaloyl chloride (8.3 g, 0.069 mol) and 4-dimethylaminopyridine (76 mg, 0.0006 mol) was added to the reaction mixture at 0°C. The reaction was stirred at 20°C for 10 hrs. Solvent was removed and the residue was washed with aqueous citric acid solution and extracted with ethyl acetate. The organic layer was washed with Na₂CO₃ and brine solution. The organic layer was dried over Na₂SO₄, concentrated in vacuum to get the crude product (13.7 g, Y= 96%). The crude material was used in the next step without further purification. HPLC-MS: Purity 80%, M+1= 226.2

### Preparation of intermediates of formula V

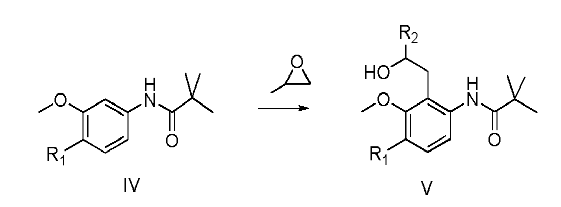

### N-(4-fluoro-2-(2-hydroxypropyl)-3-methoxyphenyl)pivalamide

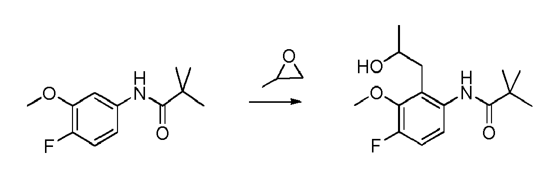

N-(4-fluoro-3-methoxyphenyl)pivalamide (2.4 g, 0.01 mol) was taken in dry tetrahydrofuran (40 ml) under nitrogen atmosphere at 0°C. Then n-BuLi (1.2 M solution in hexane, 23.3 ml, 0.028 mol) was added drop wise to the reaction mixture. The reaction mixture was stirred for 2 hr at 0°C to ensure deprotonation. Propylene oxide (1.18 ml, 0.0169 mol) was added dropwise at 0°C and stirred for an additional hour at 0°C. The reaction mixture was then allowed to reflux for 10 hr. After that, the reaction was cooled to room temperature and quenched with NH₄Cl solution. The crude was then extracted with ethyl acetate and the organic layer was washed with brine, dried over Na₂SO₄ and finally concentrated in vacuum to get the product (350 mg, Y=12%). HPLC-MS: Purity 75%, M+1= 284.3

### N-(2-(2-hydroxypropyl)-3-methoxyphenyl)pivalamide

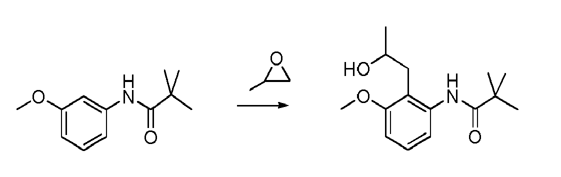

Title compound was obtained from *N*-(3-methoxyphenyl)pivalamide and propylene oxide following the synthetic procedure above described. Y=44%, HPLC-MS: Purity 80%, M+1= 266.3

### N-(2-(2-hydroxybutyl)-3-methoxyphenyl)pivalamide

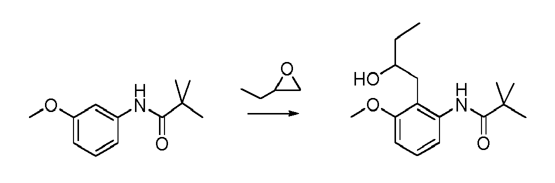

Title compound was obtained from *N*-(3-methoxyphenyl)pivalamide and 2-ethyloxirane following the synthetic procedure above described. Y=50%, HPLC-MS: Purity 88%, M+1= 280.3

### N-(2-(2-hydroxypentyl)-3-methoxyphenyl)pivalamide

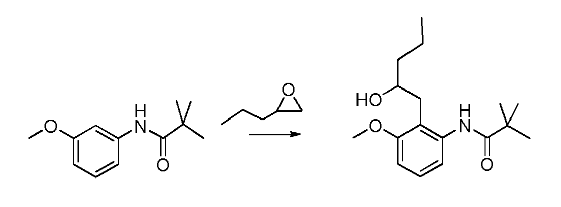

Title compound was obtained from *N*-(3-methoxyphenyl)pivalamide and 2-propyloxirane following the synthetic procedure above described. Y=30%, HPLC-MS: Purity 80%, M+1= 294.4

### Preparation of intermediates of formula VI

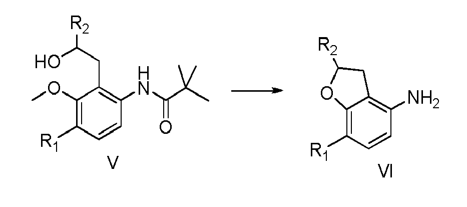

### 7-fluoro-2-methyl-2,3-dihydrobenzofuran-4-amine

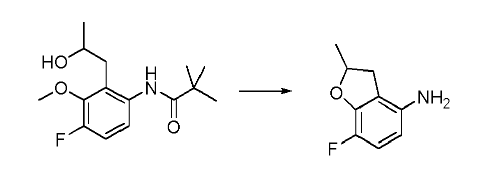

N-(4-fluoro-2-(2-hydroxypropyl)-3-methoxyphenyl)pivalamide (1 g, 3.52 mmol) and 47% HBr (10 ml) was taken in sealed tube and nitrogen was flushed for 5 min. The reaction mixture was heated at 100°C for 16 hr. After that, the reaction mixture was poured in ice-cooled water and neutralized with NaHCO₃. Ethyl acetate was added and the organic layer was separated, dried over Na₂SO₄ and concentrated in vacuum. Compound **VIa** (400 mg, Y=68%) was isolated by column chromatography. HPLC-MS: Purity 95.33%, M+1= 168.1

### 2-methyl-2,3-dihydrobenzofuran-4-amine

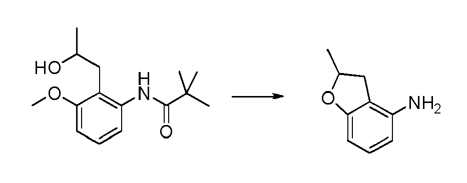

Title compound was obtained from *N*-(2-(2-hydroxypropyl)-3-methoxyphenyl)pivalamide following the synthetic procedure above described. Y=69%, HPLC-MS: Purity 93%, M+1= 150.2

### 2-ethyl-2,3-dihydrobenzofuran-4-amine

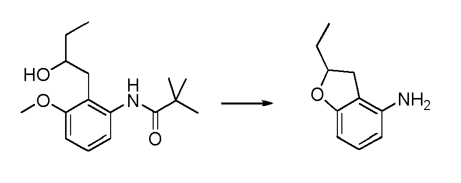

Title compound was obtained from *N*-(2-(2-hydroxybutyl)-3-methoxyphenyl)pivalamide following the synthetic procedure above described. Y=49%, HPLC-MS: Purity 90%, M+1= 164.2

### 2-propyl-2,3-dihydrobenzofuran-4-amine

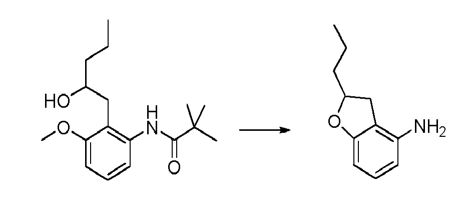

Title compound was obtained from N-(2-(2-hydroxypentyl)-3-methoxyphenyl)pivalamide following the synthetic procedure above described. Y=30%, HPLC-MS: Purity 80%, M+1= 178.2

### Preparation of intermediates of formula VII

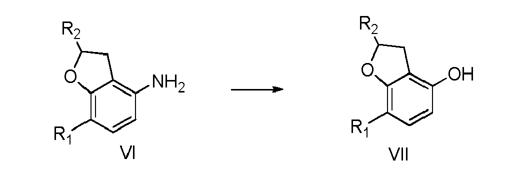

### 7-fluoro-2-methyl-2,3-dihydrobenzofuran-4-ol

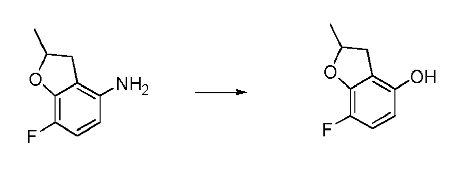

7-fluoro-2-methyl-2,3-dihydrobenzofuran-4-amine (69 mg, 0.41 mmol) was taken in HCl conc (0.18 ml) and water (5 ml). The solution was cooled to 0°C. An aqueous solution of NaNO₂ (31 mg, 0.44 mmol) was added drop wise. The reaction mixture was stirred at 100°C for 10 min and then it added drop wise to a 15 ml (1:1) mixture of HCl conc. and water, which was preheated at 100°C. The mixture was stirred for 5 additional min. at 100°C. Then it was cooled and neutralized with NaCO₃, then extracted with ethyl acetate. The organic layer dried over Na₂SO₄ and evaporated to get the final product **VIIa** (69 mg, Y=99%). HPLC-MS: Purity 95%, M+1= 169.1

### 2-methyl-2,3-dihydrobenzofuran-4-ol

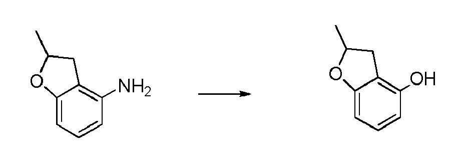

Title compound was obtained from 2-methyl-2,3-dihydrobenzofuran-4-amine following the synthetic procedure above described. Y=69%, HPLC-MS: Purity 89%, M+1= 151.17

### 2-ethyl-2,3-dihydrobenzofuran-4-ol

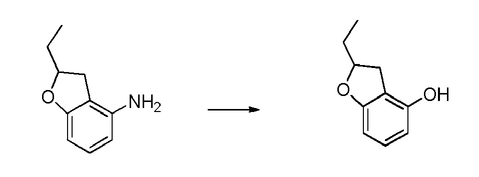

Title compound was obtained from 2-ethyl-2,3-dihydrobenzofuran-4-amine following the synthetic procedure above described. Y=65%, HPLC-MS: Purity 82%, M+1= 165.2

### 2-propyl-2,3-dihydrobenzofuran-4-ol

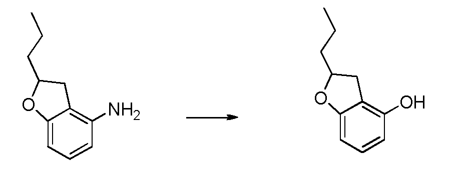

Title compound was obtained from 2-propyl-2,3-dihydrobenzofuran-4-amine following the synthetic procedure above described. Y=62%, HPLC-MS: Purity 78%, M+1= 179.2

### Preparation of intermediates of formula VIII

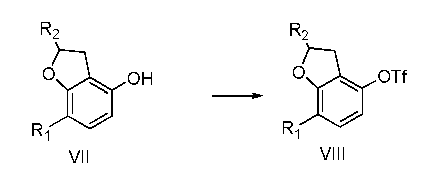

### 7-fluoro-2-methyl-2,3-dihydrobenzofuran-4-yl trifluoromethanesulfonate

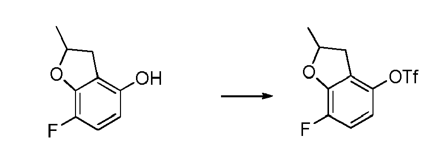

7-fluoro-2-methyl-2,3-dihydrobenzofuran-4-ol (1.2 g, 7.13 mmol) was dissolved in DCM (90 ml) under nitrogen atmosphere. Then pyridine (1.15 ml) was added to the reaction mixture at 0°C. After 10 min, triflic anhydride (1.44 ml) was added slowly to the reaction mixture and stirred for 2 hrs at 20°C. After that, water (200 ml) was added to the reaction mixture and extracted with DCM. Organic layers were dried over Na₂SO₄ and concentrated under vacuum to get the crude product (1.35 g, Y=64%). The product was used without further purification in the next synthetic step. HPLC-MS: Purity 90%, M+1= 301.2

### 2-methyl-2,3-dihydrobenzofuran-4-yl trifluoromethanesulfonate

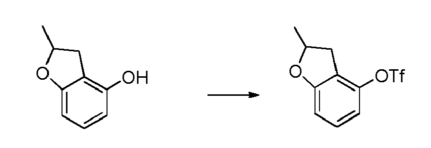

Title compound was obtained from 2-methyl-2,3-dihydrobenzofuran-4-ol following the synthetic procedure above described. Y=53%, HPLC-MS: Purity 95%, M+1= 283.2

### 2-ethyl-2,3-dihydrobenzofuran-4-yl trifluoromethanesulfonate

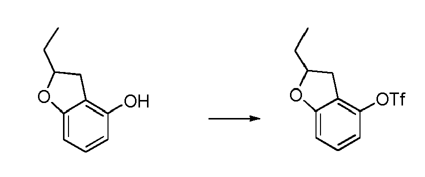

Title compound was obtained from 2-ethyl-2,3-dihydrobenzofuran-4-ol following the synthetic procedure above described. Y=60%, HPLC-MS: Purity 90%, M+1= 297.2

### 2-propyl-2,3-dihydrobenzofuran-4-yl trifluoromethanesulfonate

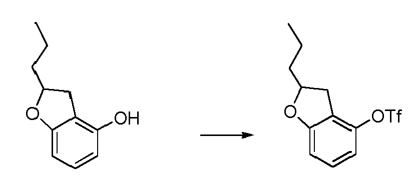

Title compound was obtained from 2-propyl-2,3-dihydrobenzofuran-4-ol following the synthetic procedure above described. Y=55%, HPLC-MS: Purity 87%, M+1= 311.3

### Preparation of intermediates of formula IX

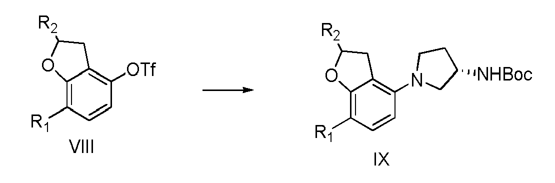

### tert-butyl (3S)-1-(7-fluoro-2-methyl-2,3-dihydrobenzofuran-4-yl)pyrrolidin-3-ylcarbamate

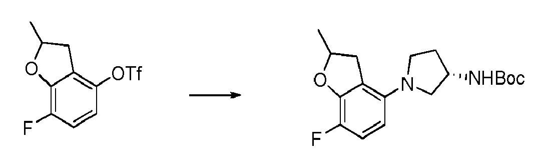

Pd(OAc)₂ (8.8 mg, 0.039 mmol) and [1,1'-binaphtthalene]-2,2'-diylbis[diphenylphosphine] (BINAP) (37 mg, 0.058 mmol) were suspended in dry toluene (7ml) under N₂ atmosphere and the mixture was degassed. Then (S)-3-(Boc-amino) pyrrolidine (60 mg, 0.326 mmol) was added to the reaction mixture. Then Cs₂CO₃ (213 mg, 0.65 mmol) was added and the mixture was degassed again. It was stirred for another 10 min and 7-fluoro-2-methyl-2,3-dihydrobenzofuran-4-yl trifluoromethane-sulfonate (98 mg, 0.32 mmol) in toluene was added and reaction mixture was refluxed overnight. After that the reaction mixture was cooled and filtered through celite. The celite bed was washed twice with EtOAc. The organic layer was then evaporated and the crude product (100 mg, Y= 91%) was purified by column chromatography. HPLC-MS: Purity 96%, M+1= 337.4

### tert-butyl (3S)-1-(2-methyl-2,3-dihydrobenzofuran-4-yl)pyrrolidin-3-ylcarbamate

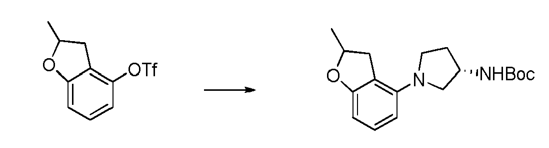

Title compound was obtained from 2-methyl-2,3-dihydrobenzofuran-4-yl trifluoromethanesulfonate following the synthetic procedure above described. Y=31 %, HPLC-MS: Purity 95%, M+1= 319.4

### tert-butyl (3S)-1-(2-ethyl-2,3-dihydrobenzofuran-4-yl)pyrrolidin-3-ylcarbamate

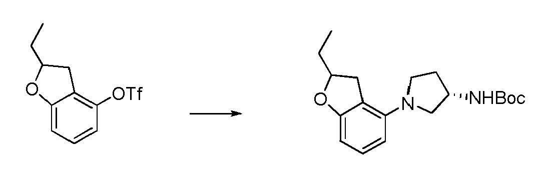

Title compound was obtained from 2-ethyl-2,3-dihydrobenzofuran-4-yl trifluoromethanesulfonate following the synthetic procedure above described. Y=30%, HPLC-MS: Purity 93%, M+1= 333.4

### tert-butyl (3S)-1-(2-propyl-2,3-dihydrobenzofuran-4-yl)pyrrolidin-3-ylcarbamate

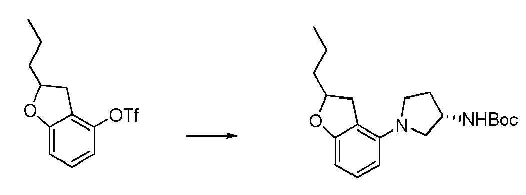

Title compound was obtained from 2-propyl-2,3-dihydrobenzofuran-4-yl trifluoromethanesulfonate following the synthetic procedure above described. Y=30%, HPLC-MS: Purity 90%, M+1= 347.4

### Preparation of intermediates of formula X

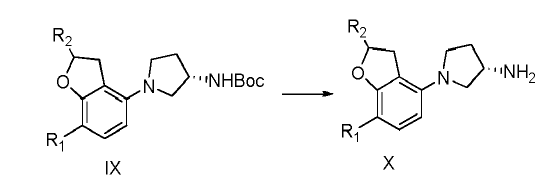

### (3S)-1-(7-fluoro-2-methyl-2,3-dihydrobenzofuran-4-yl)pyrrolidin-3-amine

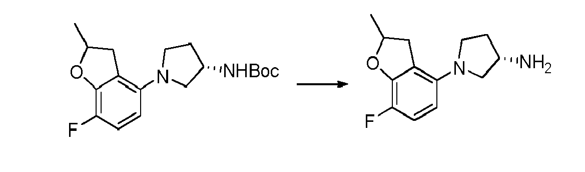

tert-butyl (3*S*)-1-(7-fluoro-2-methyl-2,3-dihydrobenzofuran-4-yl)pyrrolidin-3-ylcarbamate (1 g, 0.002 mol) was dissolved in dry DCM (20 ml). HCl gas was passed through it for 15min and then it was left under stirring for 15 min. DCM was evaporated and crude was washed with ether. Finally the crude was crystallized from DCM-hexane-ether mixture to get the product (484 mg, Y=69%). HPLC-MS: Purity 98%, M+1= 237.3

### (3S)-1-(2-methyl-2,3-dihydrobenzofuran-4-yl)pyrrolidin-3-amine

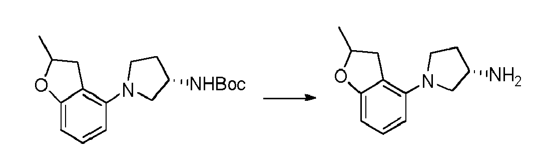

Title compound was obtained from tert-butyl (3*S*)-1-(2-methyl-2,3-dihydrobenzofuran-4-yl)pyrrolidin-3-ylcarbamate following the synthetic procedure above described. Y=100%, HPLC-MS: Purity 95%, M+1=219.3

### (3S)-1-(2-ethyl-2,3-dihydrobenzofuran-4-yl)pyrrolidin-3-amine

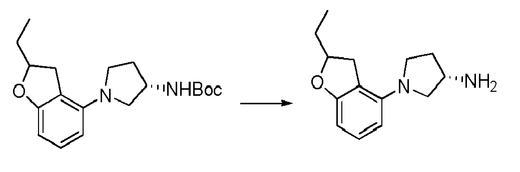

Title compound was obtained from tert-butyl (3*S*)-1-(2-ethyl-2,3-dihydrobenzofuran-4-yl)pyrrolidin-3-ylcarbamate following the synthetic procedure above described. Y=98%, HPLC-MS: Purity 95%, M+1= 233.3

### (3S)-1-(2-propyl-2,3-dihydrobenzofuran-4-yl)pyrrolidin-3-amine

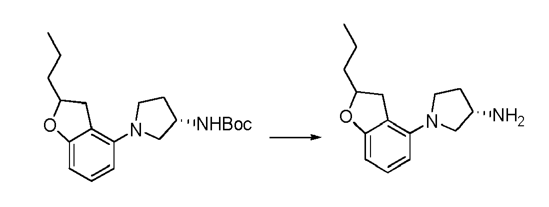

Title compound was obtained from tert-butyl (3*S*)-1-(2-propyl-2,3-dihydrobenzofuran-4-yl)pyrrolidin-3-ylcarbamate following the synthetic procedure above described. Y=85%, HPLC-MS: Purity 95%, M+1= 247.4

### EXAMPLES

### Example 1: N-((3S)-1-(7-fluoro-2-methyl-2,3-dihydrobenzofuran-4-yl)pyrrolidin-3-yl)acetamide

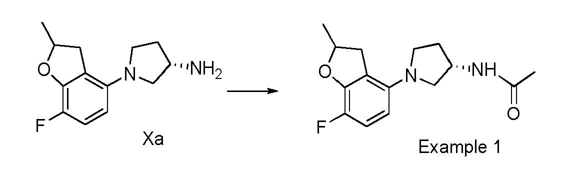

Compound **Xa** (133 mg, 0.56 mmol) was dissolved in DCM (20ml) under nitrogen atmosphere. Then Et₃N (2 ml) was added and the mixture was cooled to 0°C. After 10 min, acetyl chloride (0.04 ml, 0.56 mmol) was added. After that the reaction was stirred at 20°C for 12 hr. After that the solvent was distilled. Water was added and the mixture was extracted with DCM. The organic layer was washed with NaHCO₃, dried over Na₂SO₄, and concentrated under vacuum. The crude material was subjected to column purification to afford the final product (36 mg, Y=22%). HPLC-MS: Purity 92%, M+1= 279.3.

### Examples 2-9: N-((3S)-1-(7-fluoro-2-methyl-2,3-dihydrobenzofuran-4-yl)pyrrolidin-3-yl)-carboxamides

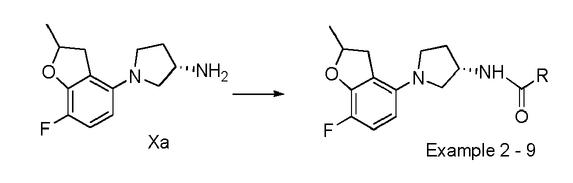

Compounds **2-9** were obtained from the appropriate acyl chloride following the synthesis described in Example 1. Results obtained are summarized in Table 2.

**Table 2. Examples 2-9**

| ***Ex.*** | ***Compound*** | ***R*** | ***Yield*** | ***HPLC-MS Purity*** | ***M+1* (*M*/*z*)** |
|---|---|---|---|---|---|
| **2** | 2,2,2-trifluoro-N-((3*S*)-1-(7-fluoro-2-methyl-2,3-dihydrobenzofuran-4-yl)pyrrolidin-3-yl)acetamide | CF₃ | 25% | 90 % | 333.3 |
| **3** | N-((3*S*)-1-(7-fluoro-2-methyl-2,3-dihydrobenzofuran-4-yl)pyrrolidin-3-yl)propionamide | Ethyl | 32% | 94% | 293.3 |
| **4** | 2-fluoro-N-((3*S*)-1-(7-fluoro-2-methyl-2,3-dihydrobenzofuran-4-yl)pyrrolidin-3-yl)propanamide | CHFCH₃ | 29% | 92% | 311.3 |
| **5** | N-((3*S*)-1-(7-fluoro-2-methyl-2,3-dihydrobenzofuran-4-yl)pyrrolidin-3-yl)butyramide | n-Pr | 32% | 94% | 307.4 |
| **6** | N-((3*S*)-1-(7-fluoro-2-methyl-2,3-dihydrobenzofuran-4-yl)pyrrolidin-3-yl)isobutyramide | i-Pr | 24% | 96% | 307.4 |
| **7** | N-((3*S*)-1-(7-fluoro-2-methyl-2,3-dihydrobenzofuran-4-yl)pyrrolidin-3-yl)cyclopropanecarboxamide | c-Pr | 27% | 91 % | 305.3 |
| **8** | N-((3*S*)-1-(7-fluoro-2-methyl-2,3-dihydrobenzofuran-4-yl)pyrrolidin-3-yl)pentanamide | n-Bu | 37% | 92% | 321.4 |
| **9** | N-((3*S*)-1-(7-fluoro-2-methyl-2,3-dihydrobenzofuran-4-yl)pyrrolidin-3-yl)-2-methylbutanamide | i-Bu | 27% | 96% | 321.4 |

### Example 10: Methyl (3S)-1-(7-fluoro-2-methyl-2,3-dihydrobenzofuran-4-yl)pyrrolidin-3-ylcarbamate

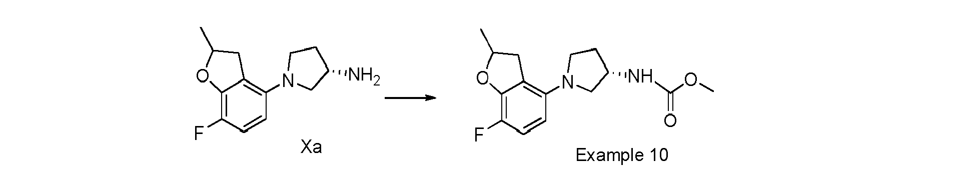

Title compound was obtained from methyl chloroformiate and intermediate **Xa** following the synthetic procedure described in Example 1. Y=28%, HPLC-MS: Purity 94 %, M+1= 295.3.

### Example 11: Ethyl (3S)-1-(7-fluoro-2-methyl-2,3-dihydrobenzofuran-4-y)pyrrolidin-3-ylcarbamate

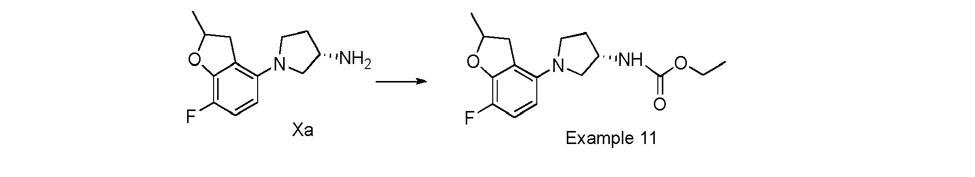

Title compound was obtained from ethyl chloroformiate and intermediate **Xa** following the synthetic procedure described in Example 1. Y=19%, HPLC-MS:. Purity 91 %, M+1= 309.35.

### Example 12: 1-Ethyl-3-((3S)-1-(7-fluoro-2-methyl-2,3-dihydrobenzofuran-4-yl)pyrrolidin-3-yl)urea

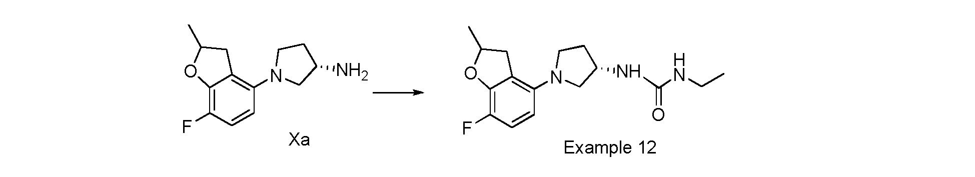

Title compound was obtained from ethyl isocianate and intermediate **Xa** following the synthetic procedure described in Example 1. Y=27%, HPLC-MS: Purity 96%, M+1= 308.3.

### Examples 13-18: N-((3S)-1-(2-methyl-2,3-dihydrobenzofuran-4-yl)-pyrrolidin-3-yl)-carboxamides

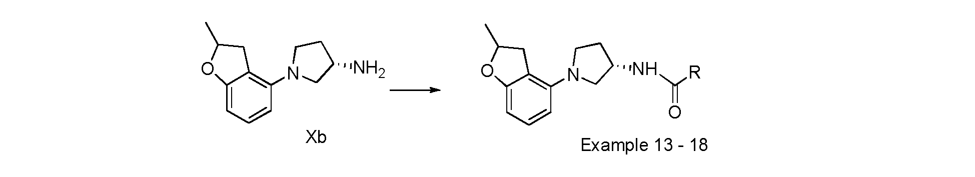

Compounds **13-18** were obtained from the appropriate acyl chloride following the synthesis described in Example 1 using (3*S*)-1-(2-methyl-2,3-dihydrobenzofuran-4-yl)pyrrolidin-3-amine instead of **Xa** as starting material. Results obtained are summarized in Table 2.

**Table 2. Examples 13-18**

| ***Ex.*** | ***Compound*** | ***R*** | ***Yield*** | ***HPLC-MS Purity* (%)** | ***M+1 (M*/*z)*** |
|---|---|---|---|---|---|
| **13** | N-((3*S*)-1-(2-methyl-2,3-dihydrobenzofuran-4-yl)pyrrolidin-3-yl)acetamide | Me | 49% | 93% | 261.3 |
| **14** | 2,2,2-trifluoro-N-((3*S*)-1-(2-methyl-2,3-dihydrobenzofuran-4-yl)pyrrolidin-3-yl)acetamide | CF₃ | 26% | 93% | 315.3 |
| **15** | N-((3*S*)-1-(2-methyl-2,3-dihydrobenzofuran-4-yl)pyrrolidin-3-yl)propionamide | Et | 56% | 95% | 275.3 |
| **16** | N-((3*S*)-1-(2-methyl-2,3-dihydrobenzofuran-4-yl)pyrrolidin-3-yl)isobutyramide | i-Pr | 32% | 93% | 289.4 |
| **17** | N-((3*S*)-1-(2-methyl-2,3-dihydrobenzofuran-4-yl)pyrrolidin-3-yl)cyclopropanecarboxamide | C-Pr | 42% | 91% | 287.4 |
| **18** | 3-methyl-N-((3*S*)-1-(2-methyl-2,3-dihydrobenzofuran-4-yl)pyrrolidin-3-yl)butanamide | i-Bu | 48% | 95% | 303.4 |

### Example 19: N-((3S)-1-(2-ethyl-2,3-dihydrobenzofuran-4-yl)pyrrolidin-3-yl)acetamide

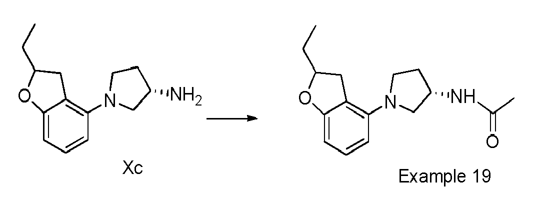

Compound **19** was obtained from the acetyl chloride following the synthesis described in Example 1 using (3*S*)-1-(2-ethyl-2,3-dihydrobenzofuran-4-yl)pyrrolidin-3-amine instead of **Xa** as starting material. Y=60%, HPLC-MS: Purity= 98%, M+1= 275.3.

### Example 20: N-((3S)-1-(2-ethyl-2,3-dihydrobenzofuran-4-yl)pyrrolidin-3-yl)propionamide

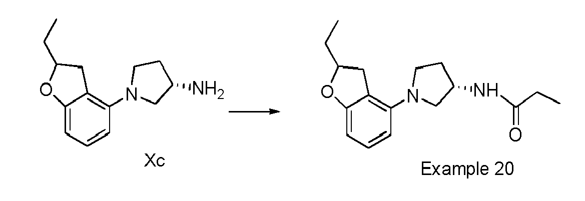

Compound **20** was obtained from the propionyl chloride following the synthesis described in Example 1 using (3*S)*-1-(2-ethyl-2,3-dihydrobenzofuran-4-yl)pyrrolidin-3-amine instead of **Xa** as starting material. Y=56%, HPLC-MS: Purity 95%, M+1= 289.3.

### Example 21: N-((3S)-1-(2-propyl-2,3-dihydrobenzofuran-4-yl)pyrrolidin-3-yl)propionamide

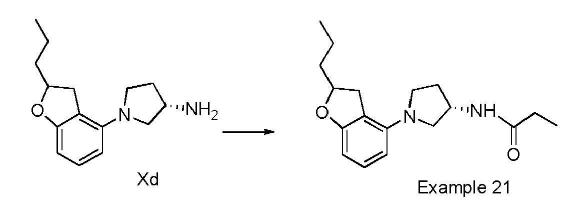

Compound **21** was obtained from the propionyl chloride following the synthesis described in Example 1 using (3S)-1-(2-propyl-2,3-dihydrobenzofuran-4-yl)pyrrolidin-3-amine instead of **Xa** as starting material. Y=31 %, HPLC-MS: Purity 94.33%, M+1= 303.4.

### Example 22: In vitro MT1 Screening

To proceed with compound screening over MT1 receptor, a cell line characterised by a stable overexpression of recombinant human-MT1 receptor was used. This line co-expresses mitochondrial apoaequorin and subunit Gα16.

Subunit Gα16 belongs to GPCR superfamily, in which intracelular signal transduction is produced via phospholipase (PLC). Activation of PLC produces an increment of inositol-triphosphate levels and intracellular calcium levels. This increment in calcium levels is, therefore, independent and full compatible with the signal transduction of MT1 receptor.

Apoaequorin is the inactive form of aequorin, a phosphoprotein that needs a prosthethic hydrophobic group, colenterazine, to achieve active forms. After binding to calcium, aequorin carries out the oxidation of colenterazine to colenteramide, a luminescent reaction.

Assay protocol for agonist screening consists in an overnight incubation of cells and colenterazine, being performed by an AequoScreen^{™} system instrument, PerkinElmer, USA. After that this mixture was injected over a plate that contains the solution of the compounds to screen and luminescence was immediately read. Only in case of antagonism screening, after 15-30min of the first injection the reference agonist was added in the same well, and then luminescence was measured.

Agonist activity was calculated as percentage of activity relative to the reference agonist at the EC₁₀₀ concentration. Table 3 summarizes the agonism % of MT1 receptor of compounds **(1-21).**

**Table 3. Agonism percentages of MT1**

| ***Compound*** | ***% MT1 at 100 nM*** | ***% MT1 at 1 nM*** |
|---|---|---|
| **Example 1** | 73 | 19 |
| **Example 2** | 79 | 32 |
| **Example 3** | 88 | 30 |
| **Example 4** | 98 | 44 |
| **Example 5** | 91 | 32 |
| **Example 6** | 75 | 24 |
| **Example 7** | 80 | 22 |
| **Example 8** | 89 | 31 |
| **Example 9** | 70 | 17 |
| **Example 10** | 89 | 36 |
| **Example 11** | 73 | 28 |
| **Example 12** | 90 | 18 |
| **Example 13** | 91 | 17 |
| **Example 14** | 79 | 8 |
| **Example 15** | 92 | 18 |
| **Example 16** | 76 | 6 |
| **Example 17** | 79 | 6 |
| **Example 18** | 82 | 28 |
| **Example 19** | 81 | 23 |
| **Example 20** | 88 | 26 |
| **Example 21** | 78 | 15 |
| *EP1189900B1, Example 1* | 98 | 27 |
| *EP1189900B1, Example 3* | 102 | 21 |
| *EP1189900B1, Example 9* | 86 | 19 |
| *Ramelteon* | 118 | 45 |
| *Melatonin* | 101 | 42 |

### Example 23: Plasma drug levels

Plasma drug levels measured at 15 and 60 min after an oral administration of 3 mg/kg in male Sprague-Dawley rats resulted in lower ratio values for the compounds of the present invention, thus showing more sustained plasma levels than prior art compounds. Data results are summarized in Table 4.

**Table 4. Plasma drug levels**

| ***Compound*** | ***R₁*** | ***R₂*** | ***R₃*** | ***n*** | ***Plasma 15min ng*/*mL*** | ***Plasma 60min ng*/*mL*** | ***Ratio 15*/*60*** |
|---|---|---|---|---|---|---|---|
| *EP1189900B1, Example 1* | H | H | Me | 0 | 467 | 120 | *3.89* |
| **Example 1** | F | Me | Me | 0 | 56.8 | 55.4 | **1.03** |
| **Example 13** | H | Me | Me | 0 | 433 | 189 | **2.29** |
| *EP1189900B1, Example 3* | H | H | Et | 0 | 215 | 105 | *2.05* |
| **Example 3** | F | Me | Et | 0 | 112.9 | 64.9 | **1.74** |
| **Example 20** | H | Et | Et | 0 | 106 | 59 | **1.80** |
| **Example 21** | H | Pr | Et | 0 | 59 | 37 | **1.59** |
| *EP1189900B1, Example 9* | H | H | cPr | 0 | 697 | 168 | *4.15* |
| **Example 7** | F | Me | cPr | 0 | 5.1 | 8.8 | **0.58** |
| **Example 17** | H | Me | cPr | 0 | 17 | 36 | **0.47** |
| *Ramelteon* | | | | | 280 | 71 | *3.94* |
| *Melatonin* | | | | | 255 | 73 | *3.49* |

In conclusion, the melatonin agonistic activity shows that the compounds of invention exhibit approximately the same activity as the reference drugs. However, it is clear that the new compounds are very useful as medicines for clinical usage because of their superior pharmaceutical effects, such as more sustained plasma levels.

## Claims

1. A 1-(2-alkyl-2,3-dihydro-benzofuran-4-yl)-pyrrolidin-3-ylamine acyl compound wherein the compound is selected from the group consisting of:
*(i)* N-((3*S*)-1-(7-fluoro-2-methyl-2,3-dihydrobenzofuran-4-yl)pyrrolidin-3-yl)acetamide;
*(ii)* 2,2,2-trifluoro-N-((3*S*)-1-(7-fluoro-2-methyl-2,3-dihydrobenzofuran-4-yl)pyrrolidin-3-yl)acetamide;
*(iii)* N-((3*S*)-1-(7-fluoro-2-methyl-2,3-dihydrobenzofuran-4-yl)pyrrolidin-3-yl)propionamide;
*(iv)* 2-fluoro-N-((3*S*)-1-(7-fluoro-2-methyl-2,3-dihydrobenzofuran-4-yl)pyrrolidin-3-yl)propanamide;
*(v)* N-((3*S*)-1-(7-fluoro-2-methyl-2,3-dihydrobenzofuran-4-yl)pyrrolidin-3-yl)butyramide;
*(vi)* N-((3*S*)-1-(7-fluoro-2-methyl-2,3-dihydrobenzofuran-4-yl)pyrrolidin-3-yl)isobutyramide;
*(vii)* N-((3*S*)-1-(7-fluoro-2-methyl-2,3-dihydrobenzofuran-4-yl)pyrrolidin-3-yl)cyclopropanecarboxamide;
*(viii)* N-((3*S*)-1-(7-fluoro-2-methyl-2,3-dihydrobenzofuran-4-yl)pyrrolidin-3-yl)pentanamide;
*(ix)* N-((3*S*)-1-(7-fluoro-2-methyl-2,3-dihydrobenzofuran-4-yl)pyrrolidin-3-yl)-2-methylbutanamide;
*(x)* methyl (3*S*)-1-(7-fiuoro-2-methyl-2,3-dihydrobenzofuran-4-yl)pyrrolidin-3-ylcarbamate;
*(xi)* ethyl (3*S*)-1-(7-fluoro-2-methyl-2,3-dihydrobenzofuran-4-yl)pyrrolidin-3-ylcarbamate;
*(xii)* 1-ethyl-3-((3*S*)-1-(7-fluoro-2-methyl-2,3-dihydrobenzofuran-4-yl)pyrrolidin-3-yl)urea;
*(xiii)* N-((3*S*)-1-(2-methyl-2,3-dihydrobenzofuran-4-yl)pyrrolidin-3-yl)acetamide;
*(xiv)* 2,2,2-trifluoro-N-((3*S*)-1-(2-methyl-2,3-dihydrobenzofuran-4-yl)pyrrolidin-3-yl)acetamide;
*(xv)* N-((3*S*)-1-(2-methyl-2,3-dihydrobenzofuran-4-yl)pyrrolidin-3-yl)propionamide;
*(xvi)* N-((3*S*)-1-(2-methyl-2,3-dihydrobenzofuran-4-yl)pyrrolidin-3-yl)isobutyramide;
*(xvii)* N-((3*S*)-1-(2-methyl-2,3-dihydrobenzofuran-4-yl)pyrrolidin-3-yl)cyclopropanecarboxamide;
*(xviii)* 3-methyl-N-((3*S*)-1-(2-methyl-2,3-dihydrobenzofuran-4-yl)pyrrolidin-3-yl)butanamide;
(xix) N-((3*S*)-1-(2-ethyl-2,3-dihydrobenzofuran-4-yl)pyrrolidin-3-yl)acetamide;
*(xx)* N-((3*S*)-1-(2-ethyl-2,3-dihydrobenzofuran-4-yl)pyrrolidin-3-yl)propionamide; and
*(xxi)* N-((3*S*)-1-(2-propyl-2,3-dihydrobenzofuran-4-yl)pyrrolidin-3-yl)propionamide; and
a pharmaceutically acceptable salt, solvate, hydrate, or an enantiomeric form thereof.

2. The use of a compound of claim 1 to prepare a medicinal product for the treatment or prevention of melatoninergic disorders.

3. The use of claim 2 wherein said melatoninergic disorders are chosen from depression, stress, sleep disorders, anxiety, seasonal affective disorders, cardiovascular pathologies, digestive system pathologies, insomnia or fatigue due to jetlag, schizophrenia, panic attacks, melancholia, appetite disorders, obesity, insomnia, psychotic disorders, epilepsy, diabetes, Parkinson's disease, senile dementia, disorders associated with normal or pathological ageing, migraine, memory loss, Alzheimer's disease and cerebral circulation disorders.

4. A pharmaceutical composition comprising a compound of claim 1 and one or more pharmaceutically acceptable excipients.

5. The use of the pharmaceutical composition of claim 4 to prepare a medicinal product for the treatment or prevention of melatoninergic disorders.

6. The use of claim 5 wherein said melatoninergic disorders are chosen from depression, stress, sleep disorders, anxiety, seasonal affective disorders, cardiovascular pathologies, digestive system pathologies, insomnia or fatigue due to jetlag, schizophrenia, panic attacks, melancholia, appetite disorders, obesity, insomnia, psychotic disorders, epilepsy, diabetes, Parkinson's disease, senile dementia, disorders associated with normal or pathological ageing, migraine, memory loss, Alzheimer's disease and cerebral circulation disorders.

7. A method of treating or preventing melatoninergic diseases that comprises administering an effective amount of one or more compounds of claim 1 to a patient.

8. A compound as defined in claim 1 for use in a method of treating or preventing melatoninergic diseases.

9. The compound of claim 8 wherein said melatoninergic disorders are chosen from depression, stress, sleep disorders, anxiety, seasonal affective disorders, cardiovascular pathologies, digestive system pathologies, insomnia or fatigue due to jetlag, schizophrenia, panic attacks, melancholia, appetite disorders, obesity, insomnia, psychotic disorders, epilepsy, diabetes, Parkinson's disease, senile dementia, disorders associated with normal or pathological ageing, migraine, memory loss, Alzheimer's disease and cerebral circulation disorders.
